# EUROPÄISCHE PATENTSCHRIFT

(11) **EP 1 190 081 B1**
(45) Veröffentlichungstag und Bekanntmachung des Hinweises auf die Patenterteilung: **28.11.2007**
(21) Anmeldenummer: 00958118.2
(22) Anmeldetag: 03.07.2000
(51) Int. Cl.: C12N 15/82, C12N 15/29, C07K 14/415, A01H 5/00

(54) **PFLANZEN MIT VERÄNDERTER GENEXPRESSION**
PLANTS WITH MODIFIED GENE EXPRESSION
PLANTES A EXPRESSION GENIQUE MODIFIEE

(30) Priorität: 02.07.1999 DE 19930570
(43) Veröffentlichungstag der Anmeldung: 27.03.2002
(73) Patentinhaber: Max-Planck-Gesellschaft zur Förderung der Wissenschaften e.V., 80539 München (DE)
(72) Erfinder: SAGASSER, Martin, D-50825 Köln (DE); WEISSHAAR, Bernd, D-50226 Frechen (DE); DEKKER, Koen, D-50829 Köln (DE)
(74) Vertreter: Dehmel, Albrecht
(86) Internationale Anmeldenummer: PCT/DE2000/002233
(87) Internationale Veröffentlichungsnummer: WO 2001/002590

(56) Entgegenhaltungen:
- WO-A-98/22604
- WO-A-98/37201
- WO-A-98/59056
- WO-A-99/00501
- WO-A-99/14351
- US-A- 5 215 912
- DATABASE EMBL SEQUENCE LIBRARY [Online] 29. März 1998 (1998-03-29) ROUNSLEY, S.D., ET AL. : "a BAC end sequence database for identifying minimal overlaps in Arabidopsis genomic sequencing. update 4 - unpublished" XP002162120
- WISMAN ELLEN ET AL: "Knock-out mutants from an En-1 mutagenized Arabidopsis thaliana population generated phenylpropanoid biosynthesis phenotypes" PROCEEDINGS OF THE NATIONAL ACADEMY OF SCIENCES OF USA,NATIONAL ACADEMY OF SCIENCE. WASHINGTON,US, Bd. 95, Nr. 21, 13. Oktober 1998 (1998-10-13), Seiten 12432-12437, XP002154815 ISSN: 0027-8424
- SHIRLEY B ET AL: "Analysis of Arabidopsis mutants deficient in flavonoid biosynthesis" PLANT JOURNAL,GB,BLACKWELL SCIENTIFIC PUBLICATIONS, OXFORD, Bd. 8, Nr. 5, 1. November 1995 (1995-11-01), Seiten 659-671, XP002082377 ISSN: 0960-7412
- DATABASE EMBL SEQUENCE LIBRARY [Online] 15. Dezember 1999 (1999-12-15) FEDERSPIEL N.A., ET AL. : "unpublished" XP002162121
- SAGASSER M ET AL: "A. thaliana TRANSPARENT TESTA 1 is involved in seed coat development and defines the WIP subfamily of plant zinc finger proteins" GENES & DEVELOPMENT, Bd. 16, 2002, Seiten 138-149,

## Beschreibung

Die vorliegende Erfindung betrifft ein Verfahren zur Herstellung von Pflanzen mit verändertem Flavonoidgehalt, umfassend das stabile Integrieren mindestens der Nukleinsäuresequenz gemäß SEQ ID NO:2 oder 4 oder einer dazu homologen Nukleinsäuresequenz in das Genom von Pflanzenzellen oder Pflanzengeweben und Regeneration der erhaltenen Pflanzenzellen oder Pflanzengeweben zu Pflanzen.

Der pflanzliche Phenylpropanoidstoffwechsel, zusammenfassend beschrieben z.B. von Weisshaar & Jenkins, Current Opinion in Plant Biology 1, 251-257 (1998) oder von Shirley, Trends in Plant Sciences 1, 377-382 (1996), besteht aus einem Netz sich verzweigender biochemischer Reaktionsketten, die die Pflanze mit den verschiedensten phenolischen Komponenten versorgen. Der identische Beginn aller nachfolgenden Synthesen geht von Phenylalanin aus und führt über Cinnamat und 4-Coumarat zu Coumaroyl-CoA. Beteiligt sind die Enzyme Phenylalanin-Ammonium-Lyase (PAL), Cinnamat-4-Hydroxylase (C4H) und 4-Coumarat-CoA-Ligase (4CL). Im weiteren Verlauf verzweigen sich die Synthesen. Eine wichtige Verzweigung führt zur Biosynthese von Flavonoiden. Flavonoide sind Flavanderivate, d.h. ausschließlich bei Pflanzen vorkommende Sekundärmetaboliten mit dem zwei aromatische Ringe beinhaltenden Flavan-Grundgerüst. Zu den vielen, oft spezifisch in einer bestimmten Pflanzenart vorkommenden Flavonoiden zählen unter anderem UV-absorbierende Flavonole, zu den Gerbstoffen gehörende Tannine und z.B. als rote und blaue Blütenfarbstoffe gebildete Anthocyane.

In *Arabidopsis thaliana* sind verschiedene chromosomale Gen-Loci bekannt, die eine Rolle in der Flavonoidbiosynthese spielen. Mutationen in etlichen dieser Loci verhindern die Akkumulation brauner Farbstoffe in der Samenschale (Testa) und werden als transparent testa (tt in mutierter Form, TT als Wildtyp) bezeichnet. Durch die unter der Samenschale liegenden Kotyledonen erscheint der Samen in diesen Fällen gelblich bis hellbraun. Wildtypsamen sind dagegen dunkelbraun. Einige dieser Loci (tt3, tt4, tt5, ttg) sind außerdem noch an der Produktion von Anthocyanen in Blättern und Sproß beteiligt und ein Locus (ttg) hat eine zusätzliche Funktion bei der Entwicklung von Trichomen und Wurzelhaaren. Alle bisher aufgetretenen tt-Mutanten haben sich als rezessiv erwiesen. Eine zusammenfassende Beschreibung findet sich in Shirley et al., The Plant Journal 8, 659-671 (1995).

Die ersten Arabidopsis-Mutanten mit Defekt in der Flavonoidbiosynthese wurden 1971 von Bürger beschrieben (Bürger, Arabidopsis Information Service 8, 36-42 (1971)). In dieser Publikation wurde der Phänotyp von tt1, der eine abweichende Samenfarbe aufweist, erstmals erwähnt. Durch genetische und morphologische Untersuchungen von Koornneef (Koornneef, Arabidopsis Information Service 18, 45-51 (1981), Koornneef, Arabidopsis Information Service 27, 1-4 (1990) sowie Shirley (Shirley et al., The Plant Journal 8, 659-671 (1995) konnte der Genlocus von tt1 auf Chromosom 1 - 54,9 sowie die Beschränkung des Phänotyps auf den Samen festgestellt werden.

Bei vielen Nutz- und Zierpflanzen ist die Veränderung der Samenzusammensetzung und eine Veränderung der Flavonoidbiosynthese aus landwirtschaftlichem und produktionstechnischen Gesichtspunkten seit langem erwünscht. Eine begrenzte Einflußnahme auf Komponenten des Flavonoidstoffwechsels war, solange die Genstruktur der beteiligten Enzyme nicht bekannt war, nur mit den Mitteln der klassischen Züchtung möglich. Diese konventionelle Methode, beruhend auf der zufälligen Vermischung des väterlichen und mütterlichen Erbguts, ist relativ zeit- und kostenintensiv. Das gewünschte Ergebnis ist erst nach 10 bis 15 Jahren zu erwarten. Die gezielte Manipulation einzelner Komponenten der Flavonoidbiosynthese ist mit klassischer Züchtung ohne genetische Analysen kaum zu erreichen. Es besteht somit die Aufgabe, Pflanzen mit einer Veränderung der Zusammensetzung des pflanzlichen Samens und einer Verbesserung der Samenqualität von Pflanzen z.B. Nutz- und Zierpflanzen bereitzustellen.

Die Aufgabe wird durch den Gegenstand der Patentansprüche gelöst.

Die Erfindung wird durch die folgenden Figuren erläutert.

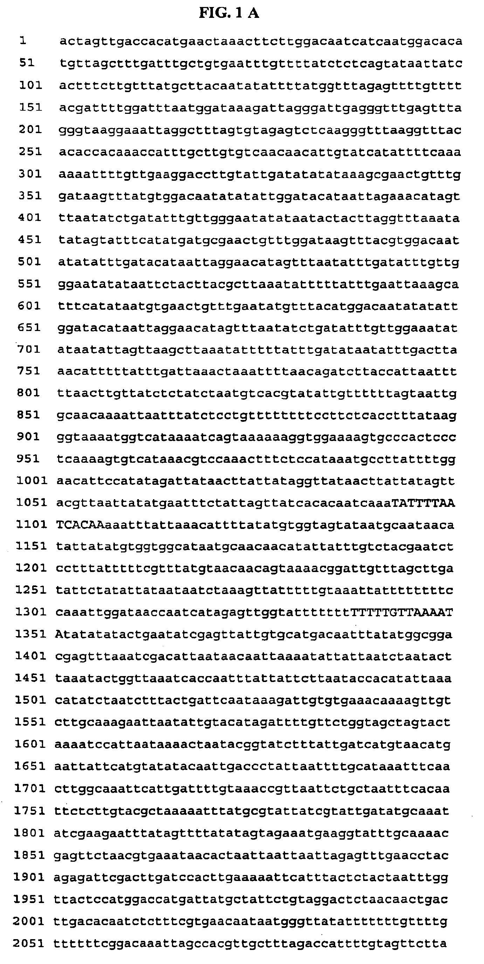

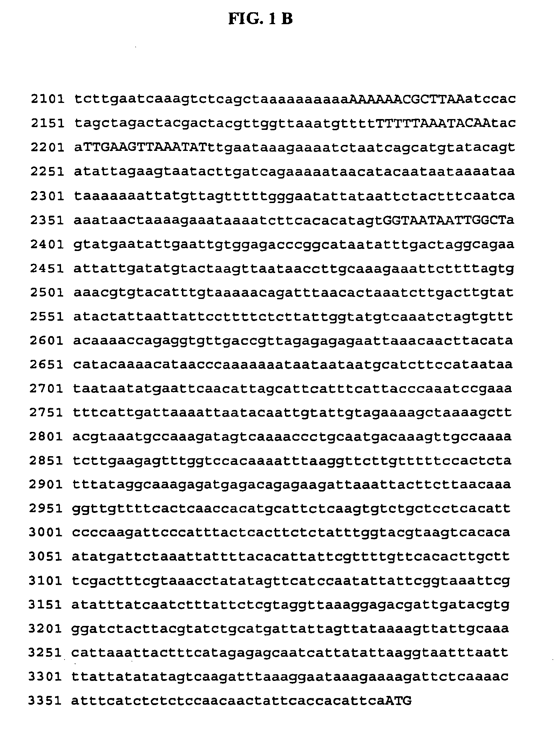

Figur 1 zeigt eine schematische Darstellung der Analyse der Nukleinsäuresequenz des TT1-Promoters durch Vergleich mit TRANSFAC MATRIX TABLE, Rel.3.3 (E. Wingender et al., 1998). Dabei sind Bindestellen für Transkriptionsfaktoren in Form von Großbuchstaben (SBF-1 like sites: siehe Lawton et al, Silencer region of a chalcone synthase promoter contains multiple binding sites for a factor, SBF-1, closely related to GT-1, Plant Molecular Biology 16, 235-249 (1991)), kursive Schrift (AGAMOUS like sites: Huang et al., Isolation and characterization of the binding sequences for the product of the Arabidopsis floral homeotic gene AGAMOUS, Nucleic Acids Research 21, 4769-4776 (1993)), unterstrichenen Buchstaben (P like sites: Grotewold et al., The myb-homologous P gene controls phlobabene pigmentation in maize floral organs by directly activating a flavonoid biosynthetic gene subset, Cell 76, 543-553 (1994)), großen und kursiven Buchstaben (MYB Ph3 like sites: Solano et al., Dual DNA binding specifity of a petal epidermis-specific MYB transcription factor (MYB.PH3) from Petunia hybrida, EMBO Journal 14, 1773-1784 (1995)) sowie großen und unterstrichenen Buchstaben (Athab-1 und 2 like sites: Sessa et al., The athb-1 and-2 HD-Zip domains homodimerize forming complexes of different DNA binding specificities, EMBO Journal 12, 3507-3517 (1993)) gekennzeichnet. Das Start-ATG ist fett und unterstrichen dargestellt. Die Numerierung beginnt mit der 5' gelegenen SpeI Schnittstelle im verwendeten Plasmidvektor pSK-TT1.

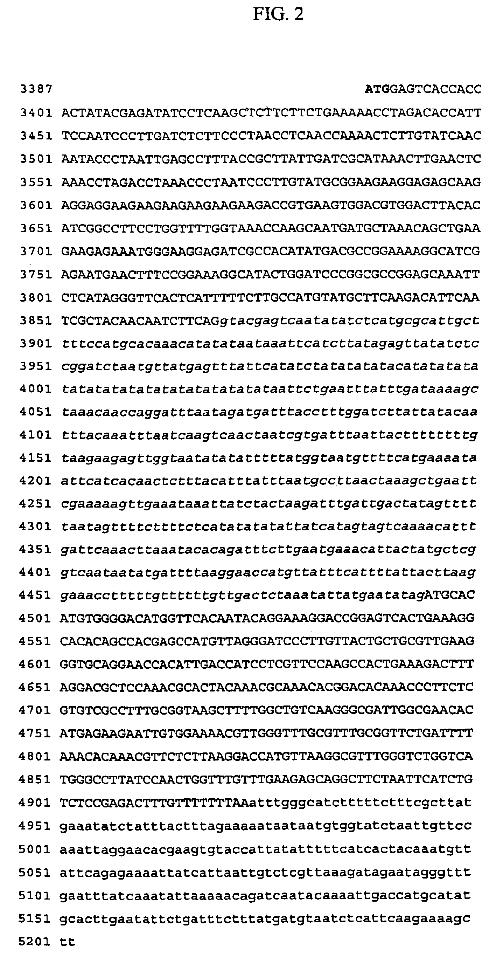

Figur 2 zeigt die Nukleinsäuresequenz der genomischen DNA-Sequenz von TT1, beginnend mit dem Start-ATG. Großbuchstaben stellen dabei Exons und kursiv geschriebene Buchstaben Introns dar. Die Numerierung setzt die Numerierung aus Figur 1 fort.

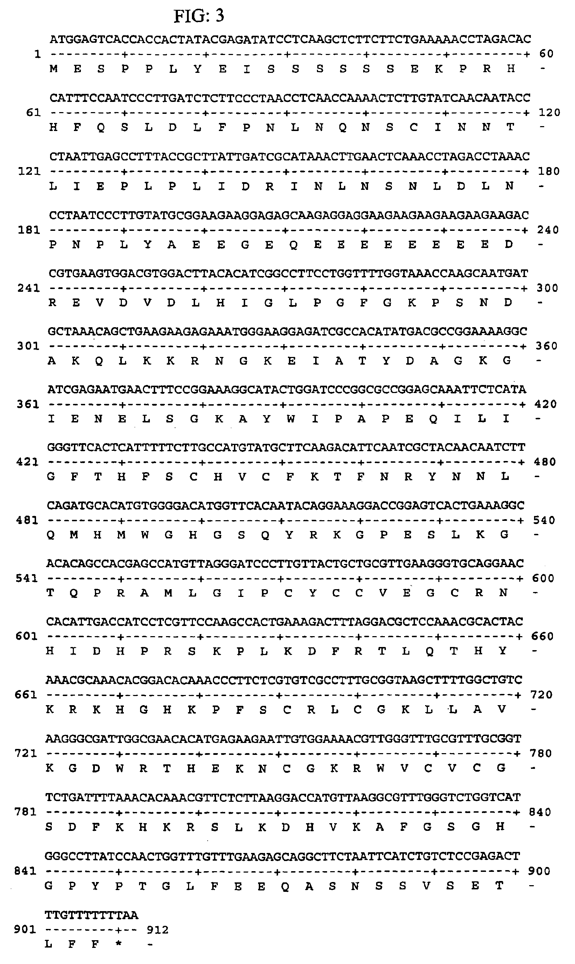

Figur 3 zeigt die für das TT1-Gen von Arabidopsis kodierende cDNA-Sequenz und die daraus abgeleitete Aminosäuresequenz von TT1.

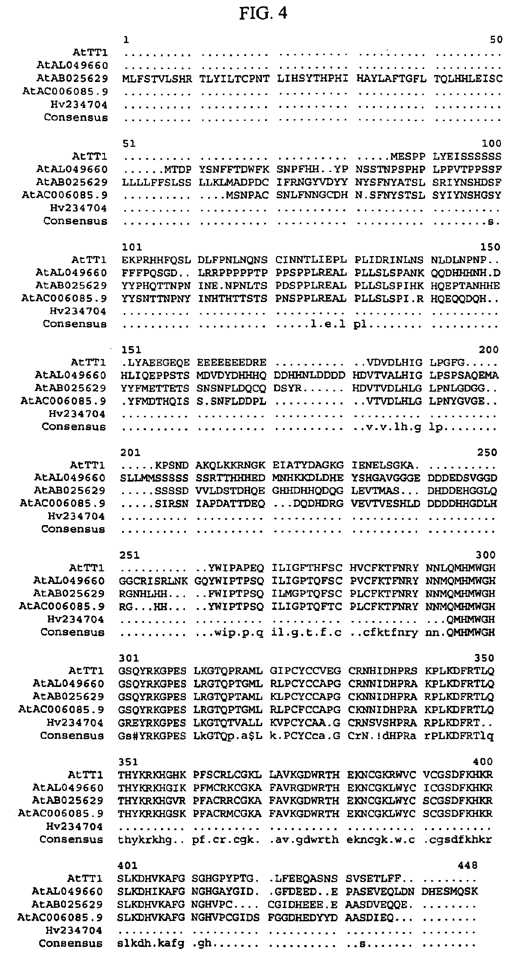

Figur 4 zeigt schematisch einen Vergleich der TT1-Aminosäuresequenz mit Sequenzen aus der NCBI GenBank. Acc.No AL049660, AB025629 und AC006085.9 sind aus Nukleinsäuresequenzen abgeleitete hypothetische Aminosäuresequenzen aus *Arabidopsis thaliana* (At), AJ234704 ist eine aus einer Nukleinsäuresequenz abgeleitete hypothetische Aminosäuresequenz für Hordeum vulgare (Hv). In der Konsensussequenz bezeichnet ein ! Aminosäuren vom Typ I oder V, ein $ Aminosäuren vom Typ L oder M, ein % Aminosäuren vom Typ F oder Y sowie ein # Aminosäuren vom Typ N, D, Q, E, B oder Z.

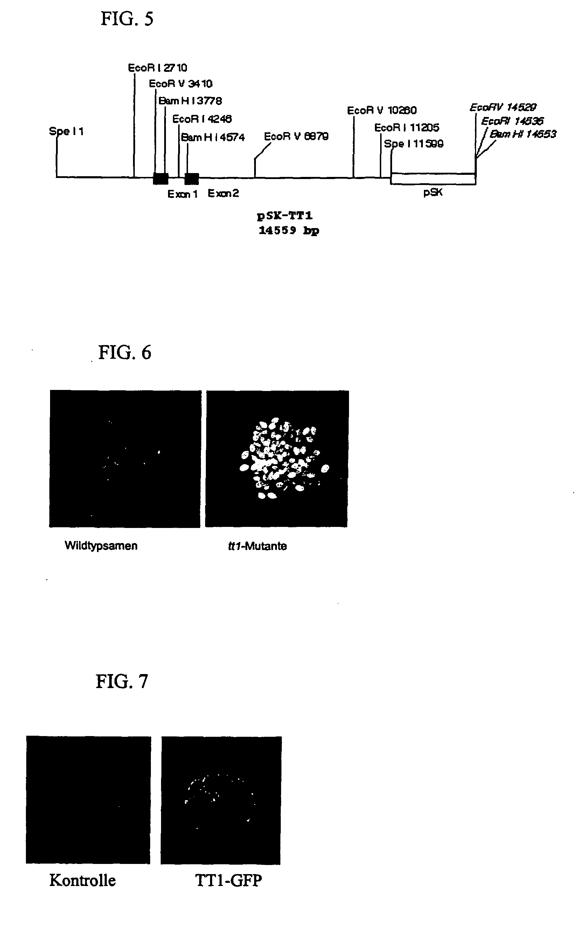

Figur 5 zeigt eine schematische Darstellung des Restriktionsmusters von pSK-TT 1.

Figur 6 zeigt eine Darstellung der Samenfärbung der Mutante tt1 im Vergleich zum Wildtyp.

Figur 7 zeigt eine Darstellung der nukleären Lokalisation von TT1.

Figur 8 zeigt X-Gluc gefärbte Blüten und Schoten transgener *TT1*-GUS-Pflanzen in verschiedenen Entwicklungsstadien. A zeigt eine Blüte kurz nach der Befruchtung. Die GUS-Aktivität ist auf die apikalen Samenanlagen beschränkt. Ältere Blüten (B) und junge Schoten (F) zeigen GUS-Aktivität in Funiculi und Integumenten apikaler und distaler Samenanlagen (C und D). Mit der verwendeten Methode ist GUS-Aktivität in Samenanlagen, die sich nicht mehr weiter entwickeln, nicht aber in älteren Samenstadien nachweisbar (E).

Der hier verwendete Ausdruck "homologe Sequenz" oder "homologe Nukleinsäuresequenz" oder "Homolog" bezeichnet eine Nukleinsäure- oder Proteinsequenz mit signifikanter Ähnlichkeit zur Vergleichssequenz, wobei diese homologen Sequenzen eine Aktivität oder Teilaktivität vergleichbar der Aktivität der Nukleinsäure- oder Proteinsequenzen gemäß SEQ ID NO:1, SEQ ID NO:2, SEQ ID NO:3 oder SEQ ID NO:4 aufweisen. Als homologe Sequenzen gelten Nukleinsäuresequehzen, die mit den Sequenzen gemäß SEQ ID NO:1, SEQ ID NO:2 oder SEQ ID NO:4 unter stringenten oder wenig stringenten Bedingungen hybridisieren (zu stringenten und wenig stringenten Bedingungen siehe Sambrook et al., Molecular Cloning, Cold Spring Harbour Laboratory (1989), ISBN 0-87969-309-6). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur. Als homologe Sequenzen sollen des weiteren Nukleinsäure- oder Proteinsequenzen gelten, die unter Zuhilfenahme des Similaritätsalgorithmus BLAST (Basic Local Alignment Search Tool, Altschul et al., Journal of Molecular Biology 215, 403-410 (1990) eine signifikante Ähnlichkeit mit den Nukleinsäure- und Aminosäuresequenzen der vorliegenden Erfindung aufweisen. Als signifikant ähnlich werden, wie hier verwendet, Sequenzen bezeichnet, die z.B. unter Verwendung von Standardparametern im Blast-Service des NCBI ein Signifikanzniveau (Probability) von P < 10⁻⁵ aufweisen, wenn Sie mit den Sequenzen gemäß SEQ ID NO:1, SEQ ID NO:2, SEQ ID NO:3 oder SEQ ID NO:4 verglichen werden.

Der hier verwendete Ausdruck "funktional verbunden" bedeutet, daß eine regulatorische Sequenz wie ein Promotor die Expression eines Gens steuert oder das eine Nukleinsäuresequenz von dem Promotor ausgehend exprimiert wird.

Der hier verwendete Ausdruck "Vektor" bezeichnet natürlich vorkommende oder künstlich erschaffene Konstrukte zur Aufnahme, Vermehrung, Expression oder Übertragung von Nukleinsäuren, z.B. Plasmide, Phagemide, Cosmide, künstliche Chromosomen, Bakteriophagen, Viren, Retroviren.

Der hier verwendete Ausdruck "Expressionssystem" bezeichnet jedwede Kombination von Vektoren, Restriktionsenzymen, Transformationsmethoden, Zellextrakten, lebenden Zellen z.B. prokaryotischen oder eukaryotischen Zellen oder Organismen mit dem Zweck der endogenen oder exogenen Expression von Genen.

Geeignete Vektoren zur Aufnahme und Überführung von regulatorischen Nukleinsäuresequenzen und mit ihnen funktional verbundenen für ein Genprodukt codierenden Nukleinsäuresequenzen können die Vermehrung und/oder die Expression der aufgenommenen Nukleinsäuren in Einzellern wie z.B. Escherichia coli oder Agrobacterium tumefaciens oder in Pflanzenzellen, Pflanzengeweben oder Pflanzen oder tierischen Zellen oder Tieren gewährleisten. Entsprechende Vektoren können natürlich vorkommen oder aber künstlich hergestellt sein. Die Vektoren können Selektionsmarker, Terminatorsequenzen, Polylinker, Promotorelemente, Enhancer, Polyadenylierungsstellen und andere genetische Elemente umfassen. Zur Klonierung geeignete Vektoren sind z.B. pBluescript, Plasmide der pUC-Serie, Plasmide der pGem-Reihe oder auf dem Bakteriophagen λ basierende Vektoren. Ein zur Verwendung in Agrobacterium benutzter Plasmidvektor ist z.B. pBinl9 (Bevan et al., Nucleic Acids Research 12, 8711-8721. (1984)). Zur Transformation und Expression in Pflanzen stellen auf dem Ti-Plasmid von Agrobacterium-Arten oder auf Pflanzenviren aufbauende Konstrukte verwendungsfähige Vektoren dar und sind dem Fachmann bekannt. Eine zusammenfassende Beschreibung bislang benutzter Vektoren findet sich in Guerineau und Mullineaux, Plant Transformation and Expression Vectors, in: Plant Molecular Biology Labfax, herausgegeben von Croy, Oxford, BIOS Scientific Publishers, 121-148 (1993).

Einige der in handelsüblichen Transformations- und Expressionssystemen angewendeten Transformationsmethoden zur Übertragung von Fremdgenen (Transformation) in das Genom von Pflanzen werden im folgenden vorgestellt. Die Wahl der Methode zur Einbringung von regulatorischen Nukleinsäuresequenzen und mit ihnen funktional verbundenen für ein Genprodukt kodierenden Nukleinsäuresequenzen in pflanzliche Zellen ist jedoch nicht auf diese Liste beschränkt. Bislang eingesetzte Transformationsverfahren bei Pflanzen sind z.B. der Gentransfer mittels Agrobacterium tumefaciens (z.B durch Baden von Samen oder Blattstückchen in einer Agrobakterienlösung), mittels pflanzlicher Viren, durch Elektroporation, durch Einschießen (microprojectile bombardment) oder Einspritzen (Mikroinjektion) sowie die Inkubation von trockenen Embryonen in DNA-haltigen Flüssigkeiten und die Transformation von Protoplasten unter Zuhilfenahme von Polyethylenglykol. Genauere Beschreibungen der angesprochenen Verfahren finden sich z.B. in Jens et al., Techniques for Gene Transfer, in: Transgenic Plants, Vol. 1, Engineering and Utilization, herausgegeben von Kung und Wu, Academic Press 128-143 (1993).

Die vorliegende Erfindung betrifft die genomische Sequenz oder die cDNA-Sequenz des TT1-Gens von *Arabidopsis thaliana* Ecotyp Columbia, das über die Bildung von Zwischenprodukten für die Bildung von Flavonoiden verantwortlich ist. Die erfindungsgemäße Nukleinsäuresequenz ist in SEQ ID NO:2 und 4 aufgeführt. Ferner betrifft die Erfindung eine Nukleinsäuresequenz, die mit der Nukleinsäuresequenz gemäß SEQ ID NO:2 oder 4 hybridisiert und für die Bildung von Flavonoiden verantwortlich ist. Bevorzugt ist eine Nukleinsäuresequenz, die unter stringenten Bedingungen mit der Nukleinsäuresequenz gemäß SEQ ID NO:2 oder 4 hybridisiert. Die vorliegende Erfindung betrifft ferner die Aminosäuresequenz des TT1-Gens von *Arabidopsis thaliana* Ecotyp Columbia gemäß SEQ ID NO:3.

Die erfindungsgemäße Nukleinsäuresequenz eignet sich z.B. zur Identifizierung und Isolierung zum TT1-Gen homologer Gene in anderen Organismen oder von homologen Genen in *Arabidopsis thaliana* mit Hilfe spezieller Hybridisierungs- oder Screening-Verfahren, z.B. als Sonde für das Screening in DNA-Bibliotheken mit Hilfe der Hybridisierung an einzelsträngige Nukleinsäuren ähnlicher Basenabfolge.

Die vorliegende Erfindung betrifft ferner ein Verfahren zur Herstellung von Organismen oder Zellen, insbesondere von Pflanzen, mit verändertem Flavonoidgehalt, umfassend das stabile Integrieren mindestens der Nukleinsäuresequenz gemäß SEQ ID NO:2 oder 4 oder einer dazu homologen Nukleinsäuresequenz in das Genom von Zellen, insbesondere von Pflanzenzellen oder Pflanzengeweben und Regeneration der erhaltenen Pflanzenzellen oder Pflanzengeweben zu Pflanzen.

Die erfindungsgemäße Nukleinsäuresequenz kann natürlichen Ursprungs sein oder aber künstlich hergestellt worden sein. Die Nukleinsäuresequenz kann sowohl in Sense- als auch in Antisense-Orientierung verwendet werden. Ferner kann die erfindungsgemäße Nukleinsäuresequenz in den genomischen Locus des TT1-Gens oder eines zum TT1-Gen homologen Gens von *Arabidopsis thaliana* oder in einen genomischen Locus eines zum TT1-Gen homologen Gens einer anderen Pflanze integriert sein. Des weiteren kann die Nukleinsäuresequenz auch in Form von Ribonukleinsäuren z.B. als Ribozym verwendet werden. In diesem Fall sind die Thymin-Basen (T) durch Uracil-Basen (U) ersetzt. Die Bildung von Flavonoiden kann durch die erfindungsgemäße Nukleinsäuresequenz sowohl verstärkt als auch verringert sein.

Die erfindungsgemäße Nukleinsäuresequenz kann z.B. zur Expression in Mikroorganismen-z.B. Escherichia coli oder Saccharomyces cerevisiae und in mono- und dikotylen Pflanzen sowie Algen und tierischen Zellen und Tieren verwendet werden. Bevorzugte Pflanzen sind dabei Kulturpflanzen z.B., Soja, Reis, Baumwolle, Zuckerrübe, Raps, Rübsen, Sonnenblume, Flachs, Hanf, Kartoffel, Tabak, Tomate, Luzerne, Salat, Erbse, Bohne, Karotte, Zwiebel, die verschiedenen Baum, Nuß- und Weinspezies, ferner Getreide wie z.B. Gerste, Weizen, Roggen, Hafer, Triticale, Mais, Futtergräser, sowie Obstsorten z.B. Mango, Apfel, Pfirsich, Stachelbeere, Johannisbeere, Banane, Melone, Kürbis und diverse Zitrusfrüchte z.B. Zitrone, Apfelsine, Pampelmuse, Mandarine. Die mit der erfindungsgemäßen Nukleinsäuresequenz transformierten Pflanzen können unmodifizierte Wildtyp-Pflanzen oder durch Züchtung erhaltene Pflanzen oder modifizierte Pflanzen z.B. transgene Pflanzen sein. Die erfindungsgemäße Nukleinsäuresequenz kann ferner nicht nur in Pflanzen, oder Pflanzengeweben sondern auch in einzelnen Pflanzenzellen z.B. in einer Zellkultur verwendet werden.

Eine Expression der erfindungsgemäßen Nukleinsäuresequenz kann durch Kombination der Sequenz mit einem geeigneten Promotor erreicht werden. Der in dieser Kombination vorliegende Promotor kann dabei sowohl der TT1-Promotor gemäß SEQ ID NO:1 als auch ein anderer endogener Promotor der transformierten Zelle oder ein auf dem Vektor befindlicher exogener Promotor sein. Als Promotor ist dabei grundsätzlich jede regulatorische Sequenz geeignet, die die Expression von Fremdgenen in Zellen, insbesondere in Pflanzen steuern kann, z.B. der CaMV 35S-Promotor aus dem Blumenkohl-Mosaik-Virus (Franck et al., Cell 21, 285-294 (1980)). Eine Expression der erfindungsgemäßen Nukleinsäuresequenz kann auch durch einen chemisch induzierbaren Promotor erreicht werden. Beispiele für chemisch induzierbare Promotoren sind der PRPI-Promotor (Ward et al., Plant Molecular Biology 22, 361-366 (1993)), ein durch Salizylsäure induzierbarer Promotor (WO 95/19443), ein durch Benzensulfonamid induzierbarer Promotor (EP-A 388186), ein durch Tetrazyklin induzierbarer Promotor (Gatz et al., Plant Journal 2, 397-404 (1992)), ein durch Abscisinsäure induzierbarer Promotor (EP-A 335528) sowie ein durch Ethanol oder Cyclohexanon induzierbarer Promotor (WO 93/21334). Je nach gewünschtem Expressionsort können auch Promotoren verwendet werden, die in bestimmten Pflanzengeweben oder Pflanzenteilen aktiv sind. Beispiele für entsprechende Promotoren sind der Phaseolin-Promotor (US 5504200), der Isoflavon-Reduktase Promotor (US 5750399), ein samenspezifischer Promotor aus Tabak (US 5824863) oder der ST-LSI Promotor aus Kartoffel (Stockhaus et al., EMBO Journal 8, 2445-2452 (1989)).

Ferner ist die erfindungsgemäße Nukleinsäuresequenz kombinierbar mit Sequenzen, die ein Targeting in bestimmte Kompartimente der Pflanze sicherstellen, z.B. für Transitpeptide oder Teile davon kodierende Sequenzen. Des weiteren sind Sequenzen, kodierend für enzymatisch aktive oder antigen wirksame Proteine z.B. His-tag mit der obengenannten erfindungsgemäßen Nukleinsäuresequenz kombinierbar.

Ferner kann die erfindungsgemäße Nukleinsäuresequenz in Vektoren, Expressionssystemen oder Pflanzen, Pflanzengeweben oder Pflanzenzellen oder tierischen Zellen oder Mikroorganismen zur Veränderung des Expressionsmusters verwendet werden.

Die erfindungsgemäße Nukleinsäuresequenz eignet sich ferner in Kombination mit verschiedenen Promotoren zur Manipulation der phänotypischen und genotypischen Eigenschaften verschiedener Pflanzen oder Pflanzengeweben, z.B. zur Veränderung der Samenfarbe, z.B. zur ästhetischen Verbesserung verschiedener Pflanzenarten. Zierpflanzen mit z.B. ausgeschaltetem oder mutiertem TT1-Gen können optisch attraktive Varietäten darstellen.

Die erfindungsgemäße Nukleinsäuresequenz kann ebenso zur Verstärkung der UV-Schutzfunktion der Samenschale durch Veränderung des Flavonoidgehaltes verwendet werden. Flavonoide zeigen Absorptionsmaxima im ultravioletten Bereich. Absorbierend wirken dabei die delokalisierten π-Elektronen der phenolischen Gruppen. Eine Erhöhung der Flavonoidkonzentration im Samen kann eine drastische Verringerung der UV-induzierten Gewebeschäden bewirken. Auf diese Weise läßt sich z.B. die Keimungsrate des Saatgutes, vor allem in Regionen mit intensiver Sonnenbestrahlung, verbessern.

Ferner kann die erfindungsgemäße Nukleinsäuresequenz zur Verstärkung der Schutzfunktion der Samenschale gegen Schädlingsbefall durch Veränderung des Flavonoidgehaltes verwendet werden. Proanthocyanidine und andere phenolische Komponenten wirken fungizid, u.a. aufgrund enzyminhibitorischer Eigenschaften (Jambunathan et al., Journal of Agricultural and Food Chemistry 34, 425-429 (1986)). Eine Konzentrierung dieser Stoffe in der Samenschale kann die Pathogenresistenz erhöhen.

Ferner kann die erfindungsgemäße Nukleinsäuresequenz zur Verbesserung der Geschmacksqualität des Samens und anderer Pflanzenteile infolge einer Veränderung des Flavonoidgehaltes, insbesondere des Gehaltes an kondensierten Tanninen, verwendet werden. Kondensierte Tannine haben einen großen Einfluß auf den Geschmack vieler Obst- und Gemüsesorten z.B. von Apfel, Kiwi oder Banane. Auf pflanzlichen Extrakten basierende Getränke z.B. Kaffee, Tee, Wein und Fruchtsäften werden ebenfalls in ihrer Geschmacksqualität von Tanninen geprägt.

Außerdem können durch den Einsatz der erfindungsgemäßen Nukleinsäuresequenz Verdaulichkeit und Nährwert sowie die Qualität der Proteinfraktion des Samens verbessert werden. Dabei kann der veränderte Flavonoidstoffwechsel die Menge und Zusammensetzung an kontaminierenden sekundären Inhaltsstoffen beeinflussen. Infolge des reduzierten Anteils an phenolischen Komponenten und antinutritiven Substanzen sind sowohl reinere als auch optisch attraktivere hellere Proteinfraktionen aus dem Samen zu gewinnen und der Rohfasergehalt kann reduziert werden.

Des weiteren kann die erfindungsgemäße Nukleinsäuresequenz aufgrund der auffälligen Gelbsamigkeit entsprechender transgener Pflanzen als optisch erkennbare genetische Markierung in der Pflanzenzucht eingesetzt werden. Insbesondere eignet sie sich als züchtungsbegleitendes Markergen zur Kennzeichnung von Pflanzen und Saatgut mit weiteren, phänotypisch nicht sichtbaren Modifikationen zur erleichterten Unterscheidbarkeit von z.B. transgenen und nichttransgenen Genotypen.

Eine weitere Verwendung der erfindungsgemäßen Nukleinsäuresequenz stellt die Verbesserung der Verarbeitungsfähigkeit des Samens in industriellen Produktionsprozessen dar. Beispielsweise verursachen kondensierte Tannine in der Testa des Samens der Gerste unerwünschte Präzipitate während des Bierbrauprozesses (Shirley, Seed Science Research 8, 415-422 (1998)). Durch reduzierte Tanningehalte kann die Präzipitatbildung verhindert werden.

Die vorliegende Erfindung betrifft ferner eine transformierte Zelle, insbesondere eine transformierte Pflanzenzelle oder ein transformiertes Pflanzengewebe, in der die erfindungsgemäße Nukleinsäuresequenz gemäß SEQ ID NO:2 oder 4 oder einer dazu homologen Nukleinsäuresequenz, stabil integriert ist. Ferner betrifft die vorliegende Erfindung eine mit der erfindungsgemäßen Nukleinsäuresequenz transformierte Pflanzenzelle oder ein transformiertes Pflanzengewebe, die oder das zu einer samenproduzierenden Pflanze regenerierbar ist. Insbesondere betrifft die vorliegende Erfindung eine Pflanze, die nach dem erfindungsgemäßen Verfahren erhältlich ist. Ferner betrifft die vorliegende Erfindung Saatgut, das von Pflanzen erhalten wird, die nach dem erfindungsgemäßen Verfahren erhalten werden.

Die vorliegende Erfindung betrifft ferner transgene Pflanzen mit einer stabil in das Genom integrierten Nukleinsäuresequenz gemäß SEQ ID NO:2 oder 4 oder einer dazu homologen Nukleinsäuresequenz, mit der biologischen Aktivität eines Polypeptids codiert durch die Nukleinsäuresequenz gemäß SEQ ID NO:2 oder 4, und gegebenenfalls einer mit dieser Nukleinsäuresequenz funktional verbundenen regulatorischen Nukleinsäuresequenz entsprechend den vorstehend aufgeführten und beschriebenen Beispielen für solche Promotoren:

### BEISPIELE

Die Erfindung wird durch die nun folgenden Beispiele erläutert, ist aber nicht auf diese beschränkt.

### Beispiel 1: Allgemeine Klonierungsverfahren

Die im Rahmen der vorliegenden Erfindung durchgeführten Klonierungsschritte wie z.B. Restriktionsspaltungen, Agarose-Gelelektrophorese, Reinigung von Nukleinsäure fragmenten, Transfer von Nukleinsäuren auf Filtermaterialien, Transformation und Anzucht von Bakterienzellen usw. wurden wie bei Sambrook et al., Molecular Cloning, Cold Spring Harbour Laboratory (1989), ISBN 0-87969-309-6, durchgeführt.

### Beispiel 2: Herstellung einer Knockout-Population von Arabidopsis thaliana

Die vorliegende Erfindung wurde durch das Screening einer mit dem Transposon En-1/Spm (Pereira et al, EMBO Journal 5, 835-841 (1986)) mutagenisierten Knockout-Population von Pflanzen der Spezies *Arabidopsis thaliana* Ecotyp Columbia erhalten. Die Integration der transposablen Elemente in Gene der Mutterpflanze führt häufig zum Ausfall der entsprechenden Genfunktion und in vielen Fällen zu einem vom Wildtyp unterscheidbaren Erscheinungsbild der betroffenen Pflanze. Zum Aufbau der Knockout-Population wurde das autonome En-1 Element aus Zea mays mittels Agrobacterium tumefaciens in Arabidopsis übertragen. Das entsprechende Transposon tagging System ist in Cardon et al., Plant Molecular Biology 23, 157-178 (1993) beschrieben. Das verwendete Ti-Plasmid, pGV3850HPT::pkEn2, beinhaltete das komplette En-1 Element als Integrat. Zur Selektion von Hygromycin-resistenten Transformanten trägt dieser Vektor das HPT-Gen unter der Kontrolle des viralen CaMV 35S Promotors. Samen einer Transformante mit einer einzelnen T-DNA-Insertion wurden auf Hygromycin-haltigem Medium ausgesät. Samen der so entstandenen, gegen Hygromycin-resistenten Pflanzen (T₂-Generation) wurden anschließend auf Kanamycin-haltigem Medium ausgesät. Bei den auf diese Weise selektionierten Pflanzen (T₃-Generation) war das En1-Element aus der T-DNA heraus transponiert. Mittels PCR wurde diejenigen Pflanzen der T₄-Generation identifiziert, die ein oder mehrere transponierte En-1 Elemente, jedoch keine En-1 Elemente mehr in der T-DNA trugen. Diese Pflanzen beinhalteten jedoch noch die T-DNAs ohne integrierte En-1 Elemente. Zur Erzeugung von En-1 positiven, T-DNA negativen Pflanzen wurde die T₄-Generation mit dem Wildtyp *Arabidopsis thaliana* Ecotyp Columbia gekreuzt und En-1 positive, T-DNA negative Pflanzen (S₀-Generation) durch PCR identifiziert. Samen jeder dieser Pflanzen wurden über 6 - 12 Generationen (bis zur S₆- bzw. S₁₂-Generation) hinweg vermehrt, bis insgesamt 3 000 Linien mit insgesamt 15 000 unabhängigen En-1 Insertionen zur Verfügung standen (Wisman et al., Plant Molecular Biology 37, 989-999 (1998), Baumann et al., Theoretical and Applied Genetics 97, 729-734 (1998)).

### Beispiel 3: Screening nach TT-Mutanten

Zur Identifizierung von phänotypisch auffälligen Mutanten der entstandenen En-1 Population wurden 2000 Familien der S₆- Generation (mit jeweils 20 Individuen) per Auge nach Auffälligkeiten durchmustert. Dabei konnte eine Linie (5K69) identifiziert werden, die sich durch eine abweichende Farbe der Samenschale (gelb statt dunkelbraun) auszeichnete, während die Produktion von Anthocyanen in Sproß und Blättern augenscheinlich nicht beeinträchtigt war.

Die Beschränkung des Phänotyps auf den Samen wurde von Koornneef, supra, und Shirley, supra, auch für die klassische ttl-Mutante beschrieben. Um zu überprüfen, ob es sich bei der in der En-Population gefundenen Linie um ein Allel von tt1 handelte, wurden beide Linien miteinander gekreuzt. Die Nachkommen dieser Kreuzung produzierten wiederum gelbe Samen. Das deutete darauf hin, daß tatsächlich beide Eltern ein defektes Allel des TT1 Gens tragen und vererbt haben.

Zur Klonierung eines Gens wurden DNA-Abschnitte bestimmt, die eine bekannte DNA-Sequenz, z.B. ein Transposon, flankieren. Dazu mußte zunächst gezeigt werden, daß sich in der Linie 5K60 das En-Transposon immer noch im TT1 Gen befand. Für diesen Zweck wurde eine Population von 51 Schwesterpflanzen der ttl-En Linie mittels Southern Blotting analysiert. 19 dieser Pflanzen produzierten gelbe und 33 dunkelbraune Samen. Es konnten verschiedene Banden identifiziert werden, die mit einer En-Sonde hybridisierten. Eine dieser Banden fand sich in allen Pflanzen, die gelbe Samen hervorbrachten, sowie in 16 der braunsamigen. Sie fehlte in den übrigen 17 Pflanzen. Dieses Ergebnis bestätigt die Annahme, daß alle gelbe Samen produzierende Pflanzen homozygot für eine Insertion des En-Transposon am tt1 Locus sind, während die braunsamigen Pflanzen entweder heterozygot für diese Insertion oder homozygot für den Wildtyp sind.

Die flankierende DNA dieser Insertion wurde durch schnelle Amplifikation genomischer Enden (RAGE), vgl. Cormack und Somssich, Gene 194, 273-276 (1997), gewonnen und in den pCR-TOPO Vektor (Firma Invitrogen) kloniert. Das resultierende Plasmid ist pCR-RAGE. Das Insert von pCR-RAGE wurde als Sonde verwendet, um die IGF-BAC Bank (Mozo et al., Plant Journal 16, 377-384 (1998)) zu durchsuchen. Dabei wurden 5 positive Klone identifiziert (1106, 3N5, 2B22, 10P4, 4M12). Alle diese Klone sind im *Arabidopsis thaliana* Genom auf Chr.I bei etwa 55cM lokalisiert, was mit der Kartenposition der tt1-Mutation übereinstimmt.

### Beispiel 4: Sequenzierung der genomischen TT1-Region

Ein 12 kb großes SpeI-Fragment des BACs 3N5, das mit der Sonde hybridisiert, wurde in pSK Bluescript (Firma Stratagene) subkloniert. Das resultierende Plasmid ist pSK-TT1. pSK-TT1 wurde unter Verwendung des Genome Priming Systems GPS 1 der Firma New England Biolabs und eines Sequenzierautomaten der Firma ABI, Modell 377, durchsequenziert. Die Sequenzen wurden zusammengefügt und mit verschiedenen Computerprogrammen untersucht. Vergleiche von TT1 mit Sequenzen aus der NCBI GenBank ergaben Ähnlichkeiten mit Zinkfingerproteinen. Zinkfingerproteine sind in der Lage, an DNA-Sequenzen zu binden und regulatorische Funktionen bezüglich der Expression bestimmter Gene auszuüben. Die von der TT1-cDNA abgeleitete Proteinsequenz von 303 AS Länge zeigt Ähnlichkeiten von etwas über 30% zu Zinkfingerproteinen wie StPCP1 (X82328, Kühn und Frommer 1995, MGG 247, 759-763) und ZmID1 (AF0058757, Colasanti et al, 1998, Cell 93, 593-603). Eine weitaus höhere Ähnlichkeit von über 70% besteht zu Datenbankeinträgen, die bislang allerdings nur hypothetische Proteine repräsentieren. Der Computervergleich zeigt neben der TT1-Aminosäuresequenz hypothethische Aminosäuresequenzen aus *Arabidopsis thaliana,* die aus den Sequenzen mit den Acc.No AL049660, AB025629 oder AC006085.9 und für Hordeum vulgare aus AJ2347041 abgeleitet wurden. Die Ähnlichkeiten erstrecken sich in diesem Fall über einen deutlich grösseren Bereich der Sequenz, gehen also über die Zinkfingerregion hinaus.

### Beispiel 5: Ermittlung der TT1-cDNA

Die Existenz eines exprimierten Gens sowie die Position des putativen Introns wurden mittels RT-PCR überprüft. Durch 3' und 5'-Race wurde die Länge der vollständigen cDNA bestimmt.

### Beispiel 6: Komplementation der tt1-Mutation

Um zu zeigen, daß es sich bei dem klonierten Gen tatsächlich um TT1 handelt, wurde die tt1 Mutation komplementiert. Dazu wurde das 12 kb Insert aus pSK-TT1 in die SpeI-Schnittstelle des Vektors pGPTV-Kan-TATA::GUS umkloniert. Dieser Vektor entstand aus pGPTV-Kan (Becker et al., Plant Molecular Biology 20, 1195-1197 (1992)) durch Austausch des CaMV 35S-Promotors gegen einen Polylinker. Nach Transformation in den Agrobakterienstamm GV3101 (mit dem Virulenzplasmid pMP90, Koncz und Schell, Molecular and General Genetics 204, 383-396 (1986)) wurden tt1-Pflanzen mittels Vakuuminfiltration transformiert (Bechthold et al., Molecular Biology and Genetics 316, 1194-1199 (1993)). Transformanten wurden auf kanamycinhaltigem MS-Medium selektiert und auf ihre Samenfarbe hin analysiert. Samen komplementierter Pflanzen entsprachen bezüglich ihrer Färbung dem Wildtyp.

### Beispiel 7: Ermittlung der zellulären Lokalisation des TT1-Proteins

Um die zelluläre Lokalisation des TT1-Proteins zu ermitteln, wurde es c-terminal an das "Grün fluoreszierende Protein" (GFP) fusioniert. Unter Verwendung von pAVA393 (von Arnim et al., Gene 221, 35-43 (1998)) und der kompletten cDNA entstand so pTT1-GFP. Das Plasmid wurde in Arabidopsis-Protoplasten transfiziert (Hartmann et al., Plant Molecular Biology 36, 741-54 (1998) und nach zwanzigstündiger Inkubation die GFP-Fluoreszenz bestimmt. Anhand dieser Untersuchung konnte die Lokalisation des TT1-Proteins im Zellkern nachgewiesen werden.

### Beispiel 8: Expressionsanalysen

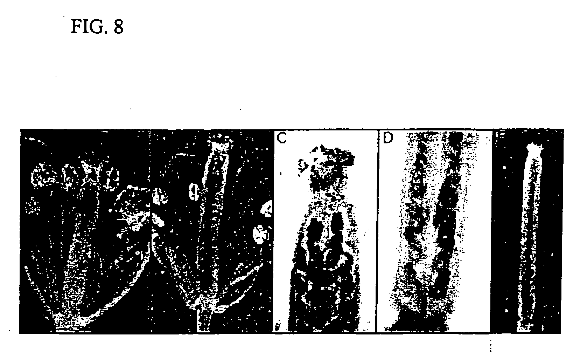

Zur Untersuchung des TT1-Promotors wurde ein 3 kb grosses Fragment zunächst in den Vektor pBT10 (Feldbrügge et al., Plant Journal 11, 1079-1093 (1997)) vor GUS gesetzt und die ganze Promotor-GUS Kassette danach in den binären Vektor pGPTV überführt. Nach Transformation in Agrobacterium wurden damit Wildtyp-Pflanzen von *Arabidopsis thaliana* Columbia infiltriert. Transformanten wurden auf kanamycinhaltigen Agarplatten selektiert. Nach etwa zehn Tagen wurden die Keimlinge auf Erde überführt und bis zur Samenreife im Gewächshaus unter Langtagbedingungen kultiviert. Die Aktivität des Reporterenzyms β-Glucuronidase wurde über die Umsetzung von farblosem X-Gluc zu einem blauen Farbstoff nachgewiesen. Dazu wurde das Substrat in Keimlinge bzw. Blätter und Infloreszenzen mit Schoten verschiedener Entwicklungsstadien infiltriert und anschließend das Chlorophyll durch ethanolische Extraktion aus dem Gewebe entfernt (Figur 8).

Nach zweitägiger Inkubation in der Substratlösung war in den transgenen *TT1*-GUS-Pflanzen die Reportergenaktivität nachweisbar. Während im Wildtyp unter diesen Bedingungen keine Blaufärbung auftrat, war in den transgenen Pflanzen übereinstimmend GUS-Aktivität in geöffneten Blüten und sich entwickelnden Schoten zu beobachten. In geöffneten Blüten überragt das Gynoeceum die Antheren (Figur 8 A und B) und die Bestäubung hat bereits stattgefunden. Die Untersuchung des Pflanzenmaterials am Lichtmikroskop ergab, daß die GUS-Aktivität in den Funiculi und Integumenten der Samenanlagen sowie in weiteren mütterlichen Geweben (Scheidewand, Schotenbasis) nachweisbar war (Figur 8 C und D). Sie wurde vor allem in den obersten und den untersten Samenanlagen junger Schoten detektiert (Figur 8 B und E). Die GUS-Aktivität konnte in Schoten bis zum Stadium 17, nicht jedoch in älteren Stadien beobachtet werden.

## Patentansprüche

1. Nukleinsäuremolekül für die Herstellung von Pflanzen mit verändertem Flavonoidgehalt, ausgewählt aus der Gruppe, bestehend aus:
a) Nukleinsäuremoleküle, die eine Nucleotidsequenz umfassen, die die in SEQ ID NO:3 angegebene Aminosäuresequenz kodiert,
b) Nukleinsäuremoleküle, die die in SEQ ID NO:2 oder 4 angegebene Nucleotidsequenz umfassen und
c) Nukleinsäuremoleküle, die eine Nucleotidsequenz umfassen, die mit einem komplementären Strang der Nucleotidsequenz von a) oder b), oder mit der Nukleinsäuresequenz gemäß a) oder b) unter stringenten Bedingungen hybridisiert, wobei die stringenten Bedingungen definiert sind als Hybridisierung in 4 x SSC bei 65°C, oder in 50% Formamid und 4 x SSC bei 42°C, gefolgt von Waschschritten in 0,1 x SSC bei 65°C für insgesamt eine Stunde.

2. Rekombinantes Nukleinsäuremolekül, umfassend
a) regulatorische Sequenzen eines in Pflanzenzellen aktiven Promotors;
b) operativ damit verknüpft eine DNA-Sequenz nach Anspruch 1;
c) optional operativ damit verknüpft regulatorische Sequenzen, die als Transkriptions-, Terminations- und/oder Polyadenylierungssignale in Pflanzenzellen dienen können.

3. Rekombinantes Nukleinsäuremolekül nach Anspruch 1 oder 2, in dem die DNA-Sequenz ausschließlich oder zusätzlich zur Sense-Orientierung in der Antisense-Orientierung vorliegt.

4. Rekombinantes Nukleinsäuremolekül nach einem der vorangehenden Ansprüche, worin der Promotor ein samenspezifischer Promotor ist.

5. Rekombinantes Nukleinsäuremolekül nach einem der vorangehenden Ansprüche, worin der Promotor ausgewählt ist aus der Gruppe der Promotoren CaMV 35S-Promotor, PRPI-Promotor, Phaseolin-Promotor, Isoflavon-Reduktase Promotor, ST-LSI Promotor, ein durch Salizylsäure induzierbarer Promotor, ein durch Benzensulfonamid induzierbarer Promotor, ein durch Tetrazyklin induzierbarer Promotor, ein durch Abscisinsäure induzierbarer Promotor, ein durch Ethanol oder Cyclohexanon induzierbarer Promotor, ein Promotor gemäß SEQ ID NO:1 oder ein samenspezifischer Promotor.

6. Vektor, umfassend ein rekombinantes Nukleinsäuremolekül nach einem der Ansprüche 1 bis 5.

7. Mikroorganismus, enthaltend ein rekombinantes Nukleinsäuremolekül oder einen Vektor nach einem der Ansprüche 1 bis 6.

8. Aminosäuresequenz, wie in SEQ ID NO:3 aufgeführt.

9. Verfahren zur Erzeugung von Pflanzen bzw. Pflanzenzellen mit einem gegenüber Wildtyppflanzen bzw. -pflanzenzellen verringerten Flavonoidgehalt, umfassend die Herstellung von Mutanten, die eine Mutation in einem homologen endogenen Gen, umfassend eine Nucleotidsequenz gemäß Anspruch 1, enthalten.

10. Verfahren zur Erzeugung von Pflanzen bzw. Pflanzenzellen mit einem gegenüber Wildtyppflanzen bzw. -pflanzenzellen verringerten Flavonoidgehalt, umfassend die folgenden Schritte:
a) Herstellung eines rekombinanten Nukleinsäuremoleküls, das Sequenzen nach einem der Ansprüche 1 bis 3 umfasst,
b) Integrieren des Nukleinsäuremoleküls aus a) in das Genom pflanzlicher Zellen; und
c) gegebenenfalls die Regeneration von Pflanzen aus den transformierten Pflanzenzellen.

11. Verfahren nach Anspruch 10, wobei die integrierte Nukleinsäuresequenz in Sense- oder Antisense-Orientierung zu der Flavonoidgehalt steuernden endogenen Nukleinsäuresequenz exprimiert wird.

12. Verfahren nach Anspruch 10 oder 11, wobei die Bildung von Flavonoiden durch ein Ribozym, umfassend die integrierte Nukleinsäuresequenz, unterdrückt wird.

13. Transgene Pflanzenzellen, enthaltend ein rekombinantes Nukleinsäuremolekül oder einen Vektor nach einem der Ansprüche 1 bis 6, oder hergestellt nach einem Verfahren gemäß einem der Ansprüche 10 bis 12.

14. Pflanzenzellen, hergestellt nach dem Verfahren gemäß Anspruch 9, die eine Mutation in einem homologen endogenen Gen, umfassend eine Nucleotidsequenz gemäß Anspruch 1, enthalten und die gegenüber dem Wildtyp einen veränderten Gehalt an Flavonoiden aufweisen.

15. Pflanzen, enthaltend eine Pflanzenzelle nach Anspruch 14 oder hergestellt nach dem Verfahren gemäß Anspruch 9, sowie Teile dieser Pflanzen, Ernteprodukte und Vermehrungsmaterial dieser Pflanzen, wie Protoplasten, Pflanzenzellen, Kalli, Samen, Knollen, Stecklinge, sowie die Nachkommen dieser Pflanzen.

16. Transgene Pflanzen, enthaltend eine Pflanzenzelle nach Anspruch 13 oder hergestellt nach einem Verfahren gemäß einem der Ansprüche 10 bis 12, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

17. Transgene Pflanzen nach Anspruch 16, wobei es sich um Ölsaaten, insbesondere Raps, handelt.

18. Pflanzen nach Anspruch 15, wobei es sich um Ölsaaten, insbesondere Raps, handelt.

19. Verwendung einer Nukleinsäuresequenz nach Anspruch 1 oder 2 zur Verringerung des Flavonoidgehalts in Pflanzen, insbesondere Rapspflanzen.

20. Verwendung nach Anspruch 19, wobei die DNA-Sequenz in Antisense-Orientierung verwendet wird.

21. Verwendung der nach einem Verfahren gemäß einem der Ansprüche 10 bis 12 hergestellten Pflanzen zur Gewinnung von Pflanzensamen mit einem im Vergleich zu Wildtyp-Pflanzensamen verringerten Gehalt an Flavonoiden.

## Claims

1. Nucleic acid molecule for producing plants with modified flavonoid content selected from the group consisting of:
a) nucleic acid molecules comprising a nucleotide sequence encoding the amino acid sequence according to SEQ ID NO:3,
b) nucleic acid molecules comprising the nucleotide sequence according to SEQ ID NO:2 or 4, and
c) nucleic acid molecules comprising a nucleotide sequence, which hybridizes to a complementary strand of the nucleotide sequence of a) or b), or with the nucleic acid sequence according to a) or b) under stringent conditions, wherein the stringent conditions are defined to hybridization in 4 x SSC at 65°C, or in 50% formamide and 4 x SSC at 42°C, followed by washing steps in 0,1 x SSC at 65°C for at large one hour.

2. Recombinant nucleic acid molecule, comprising:
a) regulatory sequences of a promoter active in plant cells;
b) a DNA-sequence according to claim 1 operatively linked therewith;
c) optionally regulatory sequences operatively linked therewith, which can conduce to transcription, termination, and/or polyadenylation signals in plant cells.

3. Recombinant nucleic acid molecule according to claim 1 or 2 having a DNA sequence exclusively or in addition in the antisense orientation to the sense orientation.

4. Recombinant nucleic acid molecule according to anyone of the preceding claims, wherein the promoter is a seed-specific promoter.

5. Recombinant nucleic acid molecule according to anyone of the preceding claims, wherein the promoter is selected from the group of promoters CaMV 35S Promoter, PRPI promoter, phaseolin promoter, isoflavon reductase promoter, ST-LSI promoter, salicylic acid-inducible promoter, benzenesulfonamide-inducible promoter, tetracycline-inducible promoter, abscisic acid-inducible promoter, ethanol- or cyclohexanon-inducible promoter, a promoter according to SEQ ID NO:1 or a seed specific promoter.

6. Vector comprising a recombinant nucleic acid molecule according to anyone of claims 1 to 5.

7. Microorganism containing a recombinant nucleic acid molecule or a vector according to anyone of claims 1 to 6.

8. Amino acid sequence according to SEQ ID NO:3.

9. Method for producing plants and plant cells, respectively, with a reduced flavonoid content compared to wild-type plants and plant cells, respectively, comprising the production of mutants having a mutation in a homologous endogenous gene comprising a nucleotide sequence according to claim 1.

10. Method for producing plants and plant cells, respectively, with a reduced flavonoid content compared to wild-type plants and plant cells, respectively, comprising the following steps:
a) producing a recombinant nucleic acid molecule comprising sequences according to anyone of claims 1 to 3,
b) integrating said nucleic acid molecule of step a) into the genome of plant cells; and
c) optionally regenerating of plants of said transformed plant cells.

11. Method according to claim 10, wherein the integrated nucleic acid sequence is expressed in sense or antisense orientation to the endogenous nucleic acid sequence affecting the flavonoid content.

12. Method according to claim 10 or 11, wherein the formation of flavonoids is inhibited by a ribozyme comprising the integrated nucleic acid sequence.

13. Transgenic plant cells, containing a recombinant nucleic acid molecule or a vector according to anyone of claims 1 to 6, or produced according to a method according to anyone of claims 10 to 12.

14. Plant cells produced according to the method of claim 9, containing a mutation in a homologous endogenous gene comprising a nucleotide sequence according to claim 1 and having a modified flavonoid content compared to said wild type.

15. Plants containing a plant cell according to claim 14 or produced according to the method of claim 9, as well as parts of said plants, harvest products and reproduction material of said plants such as protoplasts, plant cells, calli, seeds, tubers, cuttings, as well as progeny of said plants.

16. Transgenic plants, containing a plant cell according to claim 13 or produced according to anyone of the methods of claims 10 to 12, as well as parts of said plants, transgenic harvest products and transgenic reproduction material of said plants, such as protoplasts, plant cells, calli, seeds, tubers, cuttings, as well as transgenic progeny of said plants.

17. Transgenic plants according to claim 16, wherein said transgenic plants are oilseeds, in particular colza.

18. Plants according to claim 15, wherein said plants are oilseeds, in particular colza.

19. Use of a nucleic acid sequence according to claim 1 or 2 for reducing the flavonoid content in plants, in particular colza plants.

20. Use according to claim 19, wherein the DNA sequence is used in antisense orientation.

21. Use of plants produced according to a method of anyone of claims 10 to 12 for the production of plant seeds with a reduced flavonoid content compared to wild-type plant seeds.

## Revendications

1. Molécule d'acide nucléique pour la production de végétaux ayant une teneur en flavonoïdes modifiée, choisie dans le groupe constitué par :
a) les molécules d'acide nucléique qui comprennent une séquence nucléotidique qui code la séquence d'acides aminés indiquée en SEQ ID N°3,
b) les molécules d'acide nucléique qui comprennent une séquence nucléotidique indiquée en SEQ ID N°2 ou 4 et
c) les molécules d'acide nucléique qui comprennent une séquence nucléotidique qui s'hybride avec un brin complémentaire de la séquence nucléotidique de a) ou de b), ou avec la séquence d'acide nucléique selon a) ou b) dans des conditions stringentes, où les conditions stringentes sont définies comme étant l'hybridation dans 4 x SSC à 65°C, ou dans 50 % de formamide et 4 x SSC à 42°C, suivie par des étapes de lavage dans 0,1 x SSC à 65°C pour au total une heure.

2. Molécule d'acide nucléique recombinante, comprenant
a) des séquences régulatrices d'un promoteur actif dans les cellules végétales ;
b) liée de manière fonctionnelle à celle-ci, une séquence d'ADN selon la revendication 1 ;
c) le cas échéant liée de manière fonctionnelle à celle-ci, des séquences régulatrices qui peuvent servir de signaux de transcription, de terminaison et/ou de polyadénylation dans des cellules végétales.

3. Molécule d'acide nucléique recombinante selon la revendication 1 ou 2, dans laquelle la séquence d'ADN est présente exclusivement, ou bien en plus de l'orientation sens, dans l'orientation antisens.

4. Molécule d'acide nucléique recombinante selon l'une des revendications précédentes, dans laquelle le promoteur est un promoteur spécifique des graines.

5. Molécule d'acide nucléique recombinante selon l'une des revendications précédentes, dans laquelle le promoteur est choisi dans le groupe constitué par les promoteurs : promoteur CaMV 35S, promoteur PRPI, promoteur de la phaséoline, promoteur de la réductase d'isoflavone, promoteur de ST-LSI, un promoteur inductible par l'acide salicylique, un promoteur inductible par le benzènesulfonamide, un promoteur inductible par la tétracycline, un promoteur inductible par l'acide abscisique, un promoteur inductible par l'éthanol ou la cyclohexanone, un promoteur selon la SEQ ID N°1 ou un promoteur spécifique à des graines.

6. Vecteur comprenant une molécule d'acide nucléique recombinante selon l'une des revendications 1 à 5.

7. Microorganisme contenant une molécule d'acide nucléique recombinante ou un vecteur selon l'une des revendications 1 à 6.

8. Séquence d'acides aminés telle qu'elle est mentionnée dans la SEQ ID N°3.

9. Procédé de production de végétaux ou de cellules végétales avec une teneur en flavonoïdes réduite par rapport aux végétaux ou cellules végétales de type sauvage, comprenant la production de mutants, qui contiennent une mutation dans un gène endogène homologue, comprenant une séquence nucléotidique selon la revendication 1.

10. Procédé de production de végétaux ou de cellules végétales avec une teneur en flavonoïdes réduite par rapport aux végétaux ou cellules végétales de type sauvage, comprenant les étapes suivantes :
a) production d'une molécule d'acide nucléique recombinante, qui comprend des séquences selon l'une des revendications 1 à 3,
b) intégration de la molécule d'acide nucléique provenant de l'étape a) dans le génome des cellules végétales ; et
c) le cas échéant, régénération des végétaux à partir des cellules végétales transformées.

11. Procédé selon la revendication 10, dans lequel la séquence d'acide nucléique intégrée est exprimée dans l'orientation sens ou antisens par rapport à la séquence d'acide nucléique endogène régulant la teneur en flavonoïdes.

12. Procédé selon la revendication 10 ou 11, dans lequel la formation des flavonoïdes est réprimée par un ribozyme, comprenant la séquence d'acide nucléique intégrée.

13. Cellules végétales transgéniques, contenant une molécule d'acide nucléique recombinante ou un vecteur selon l'une des revendications 1 à 6, ou produites d'après un procédé selon l'une des revendications 10 à 12.

14. Cellules végétales, produites d'après le procédé selon la revendication 9, qui contiennent une mutation dans un gène endogène homologue, comprenant une séquence nucléotidique selon la revendication 1, et qui présentent une teneur modifiée en flavonoïdes par rapport au type sauvage.

15. Végétaux contenant une cellule végétale d'après la revendication 14 ou produits d'après le procédé selon la revendication 9, ainsi que parties de ces végétaux, produits de la récolte et matériel de reproduction de ces végétaux, comme les protoplastes, les cellules végétales, les cals, les graines, les tubercules, les plantules, ainsi que les rejetons de ces végétaux.

16. Végétaux transgéniques, contenant une cellule végétale d'après la revendication 13 ou produits d'après un procédé selon l'une des revendications 10 à 12, ainsi que parties de ces végétaux, produits transgéniques de la récolte et matériel de reproduction transgénique de ces végétaux, comme les protoplastes, les cellules végétales, les cals, les graines, les tubercules, les plantules, ainsi que les rejetons transgéniques de ces végétaux.

17. Végétaux transgéniques selon la revendication 16, qui sont des graines d'oléagineux, en particulier de colza.

18. Végétaux selon la revendication 15, qui sont des graines d'oléagineux, en particulier de colza.

19. Utilisation d'une séquence d'acide nucléique selon la revendication 1 ou 2 pour réduire la teneur en flavonoïdes dans les végétaux, en particulier les plants de colza.

20. Utilisation selon la revendication 19, dans laquelle la séquence d'ADN est utilisée dans l'orientation antisens.

21. Utilisation des végétaux produits d'après un procédé selon l'une des revendications 10 à 12 pour l'obtention de graines de végétaux ayant une teneur en flavonoïdes réduite par rapport aux graines de végétaux de type sauvage.


### SEQUENZPROTOKOLL

<110> Max-Planck-Gesellschaft z. Förd. d. Wissenschaften
<120> Pflanzen mit veränderter Genexpression
<130> GI-001
<140> xx
   <141> 1999-07-02
<160> 4
<170> PatentIn Ver. 2.1
<210> 1
   <211> 3389
   <212> DNA
   <213> *Arabidopsis thaliana*
<400> 1
   actagttgac cacatgaact aaacttcttg gacaatcatc aatggacaca tgttagcttt 60
   gatttgctgt gaatttgttt tatctctcag tataattatc actttcttgt ttatgcttac 120
   aatatatttt atggtttaga gttttgtttt acgattttgg atttaatgga taaagattag 180
   ggattgaggg tttgagttta gggtaaggaa attaggcttt agtgtagagt ctcaagggtt 240
   taaggtttac acaccacaaa ccatttgctt gtgtcaacaa cattgtatca tattttcaaa 300
   aaaattttgt tgaaggacct tgtattgata tatataaagc gaactgtttg gataagttta 360
   tgtggacaat atatattgga tacataatta gaaacatagt ttaatatctg atatttgttg 420
   ggaatatata atactactta ggtttaaata tatagtattt catatgatgc gaactgtttg 480
   gataagttta cgtggacaat atatatttga tacataatta ggaacatagt ttaatatttg 540
   atatttgttg ggaatatata attctactta cgcttaaata tttttatttg aattaaagca 600
   tttcatataa tgtgaactgt ttgaatatgt ttacatggac aatatatatt ggatacataa 660
   ttaggaacat agtttaatat ctgatatttg ttggaaatat ataatattag ttaagcttaa 720
   atatttttat ttgatataat atttgactta aacattttta tttgattaaa ctaaatttta 780
   acagatctta ccattaattt ttaacttgtt atctctatct aatgtcacgt atattgtttt 840
   ttagtaattg gcaacaaaat taatttatct cctgtttttt ttccttctca cctttataag 900
   ggtaaaatgg tcataaaatc agtaaaaaag gtggaaaagt gcccactccc tcaaaagtgt 960
   cataaacgtc caaactttct ccataaatgc cttattttgg aacattccat atagattata 1020
   acttattata ggttataact tattatagtt acgttaatta tatgaatttc tattagttat 1080
   cacacaatca aatattttaa tcacaaaaat ttattaaaca ttttatatgt ggtagtataa 1140
   tgcaataaca tattatatgt ggtggcataa tgcaacaaca tattatttgt ctacgaatct 1200
   cctttatttt tcgtttatgt aacaacagta aaacggattg tttagcttga tattctatat 1260
   tataataatc taaagttatt tttgtaaatt attttttttc caaattggat aaccaatcat 1320
   agagttggta tttttttttt ttgttaaaat atatatatac tgaatatcga gttattgtgc 1380
   atgacaattt atatggcgga cgagtttaaa tcgacattaa taacaattaa aatattatta 1440
   atctaatact taaatactgg ttaaatcacc aatttattat tcttaatacc acatattaaa 1500
   catatctaat ctttactgat tcaataaaga ttgtgtgaaa caaaagttgt cttgcaaaga 1560
   attaatattg tacatagatt ttgttctggt agctagtact aaaatccatt aataaaacta 1620
   atacggtatc tttattgatc atgtaacatg aattattcat gtatatacaa ttgaccctat 1680
   taattttgca taaatttcaa cttggcaaat tcattgattt tgtaaaccgt taattctgct 1740
   aatttcacaa ttctcttgta cgctaaaaat ttatgcgtat tatcgtattg atatgcaaat 1800
   atcgaagaat ttatagtttt atatagtaga aatgaaggta tttgcaaaac gagttctaac 1860
   gtgaaataac actaattaat taattagagt ttgaacctac agagattcga cttgatccac 1920
   ttgaaaaatt catttactct actaatttgg ttactccatg gaccatgatt atgctattct 1980
   gtaggactct aacaactgac ttgacacaat ctctttcgtg aacaataatg ggttatattt 2040
   ttttgttttg ttttttcgga caaattagcc acgttgcttt agaccatttt gtagttctta 2100
   tcttgaatca aagtctcagc taaaaaaaaa aaaaaaacgc ttaaatccac tagctagact 2160
   acgactacgt tggttaaatg ttttttttta aatacaatac attgaagtta aatatttgaa 2220
   taaagaaaat ctaatcagca tgtatacagt atattagaag taatacttga tcagaaaaat 2280
   aacatacaat aataaaataa taaaaaaatt atgttagttt ttgggaatat tataattcta 2340
   ctttcaatca aaataactaa aagaaataaa atcttcacac atagtggtaa taattggcta 2400
   gtatgaatat tgaattgtgg agacccggca taatatttga ctaggcagaa attattgata 2460
   tgtactaagt taataacctt gcaaagaaat tcttttagtg aaacgtgtac atttgtaaaa 2520
   acagatttaa cactaaatct tgacttgtat atactattaa ttattccttt tctcttattg 2580
   gtatgtcaaa tctagtgttt acaaaaccag aggtgttgac cgttagagag agaattaaac 2640
   aacttacata catacaaaac ataacccaaa aaaataataa taatgcatct tccataataa 2700
   taataatatg aattcaacat tagcattcat ttcattaccc aaatccgaaa tttcattgat 2760
   taaaattaat acaattgtat tgtagaaaag ctaaaagctt acgtaaatgc caaagatagt 2820
   caaaaccctg caatgacaaa gttgccaaaa tcttgaagag tttggtccac aaaatttaag 2880
   gttcttgttt ttccactcta tttataggca aagagatgag acagagaaga ttaaattact 2940
   tcttaacaaa ggttgttttc actcaaccac atgcattctc aagtgtctgc tcctcacatt 3000
   ccccaagatt cccatttact cacttctcta tttggtacgt aagtcacaca atatgattct 3060
   aaattatttt acacattatt cgttttgttc acacttgctt tcgactttcg taaacctata 3120
   tagttcatcc aatattattc ggtaaattcg atatttatca atctttattc tcgtaggtta 3180
   aaggagacga ttgatacgtg ggatctactt acgtatctgc atgattatta gttataaaag 3240
   ttattgcaaa cattaaatta ctttcataga gagcaatcat tatattaagg taatttaatt 3300
   ttattatata tagtcaagat ttaaaggaat aaagaaaaga ttctcaaaac atttcatctc 3360
   tctccaacaa ctattcacca cattcaatg                                   3389
<210> 2
   <211> 912
   <212> DNA
   <213> *Arabidopsis thaliana*
<400> 2
   atggagtcac caccactata cgagatatcc tcaagctctt cttctgaaaa acctagacac 60
   catttccaat cccttgatct cttccctaac ctcaaccaaa actcttgtat caacaatacc 120
   ctaattgagc ctttaccgct tattgatcgc ataaacttga actcaaacct agacctaaac 180
   cctaatccct tgtatgcgga agaaggagag caagaggagg aagaagaaga agaagaagac 240
   cgtgaagtgg acgtggactt acacatcggc cttcctggtt ttggtaaacc aagcaatgat 300
   gctaaacagc tgaagaagag aaatgggaag gagatcgcca catatgacgc cggaaaaggc 360
   atcgagaatg aactttccgg aaaggcatac tggatcccgg cgccggagca aattctcata 420
   gggttcactc atttttcttg ccatgtatgc ttcaagacat tcaatcgcta caacaatctt 480
   cagatgcaca tgtggggaca tggttcacaa tacaggaaag gaccggagtc actgaaaggc 540
   acacagccac gagccatgtt agggatccct tgttactgct gcgttgaagg gtgcaggaac 600
   cacattgacc atcctcgttc caagccactg aaagacttta ggacgctcca aacgcactac 660
   aaacgcaaac acggacacaa acccttctcg tgtcgccttt gcggtaagct tttggctgtc 720
   aagggcgatt ggcgaacaca tgagaagaat tgtggaaaac gttgggtttg cgtttgcggt 780
   tctgatttta aacacaaacg ttctcttaag gaccatgtta aggcgtttgg gtctggtcat 840
   gggccttatc caactggttt gtttgaagag caggcttcta attcatctgt ctccgagact 900
   ttgttttttt aa                                                     912
<210> 3
   <211> 303
   <212> PRT
   <213> *Arabidopsis thaliana*
<400> 3
   Met Glu Ser Pro Pro Leu Tyr Glu Ile Ser Ser Ser Ser Ser Ser Glu
    1               5                  10                  15
   Lys Pro Arg His His Phe Gln Ser Leu Asp Leu Phe Pro Asn Leu Asn
               20                  25                  30
   Gln Asn Ser Cys Ile Asn Asn Thr Leu Ile Glu Pro Leu Pro Leu Ile
           35                  40                  45
   Asp Arg Ile Asn Leu Asn Ser Asn Leu Asp Leu Asn Pro Asn Pro Leu
       50                  55                  60
   Tyr Ala Glu Glu Gly Glu Gln Glu Glu Glu Glu Glu Glu Glu Glu Asp
   65                  70                  75                  80
   Arg Glu Val Asp Val Asp Leu His Ile Gly Leu Pro Gly Phe Gly Lys
                   85                  90                  95
   Pro Ser Asn Asp Ala Lys Gln Leu Lys Lys Arg Asn Gly Lys Glu Ile
               100                 105                 110
   Ala Thr Tyr Asp Ala Gly Lys Gly Ile Glu Asn Glu Leu Ser Gly Lys
           115                 120                 125
   Ala Tyr Trp Ile Pro Ala Pro Glu Gln Ile Leu Ile Gly Phe Thr His
       130                 135                 140
   Phe Ser Cys His Val Cys Phe Lys Thr Phe Asn Arg Tyr Asn Asn Leu
   145                 150                 155                 160
   Gln Met His Met Trp Gly His Gly Ser Gln Tyr Arg Lys Gly Pro Glu
                   165                 170                 175
   Ser Leu Lys Gly Thr Gln Pro Arg Ala Met Leu Gly Ile Pro Cys Tyr
               180                 185                 190
   Cys Cys Val Glu Gly Cys Arg Asn His Ile Asp His Pro Arg Ser Lys
           195                 200                 205
   Pro Leu Lys Asp Phe Arg Thr Leu Gln Thr His Tyr Lys Arg Lys His
       210                 215                 220
   Gly His Lys Pro Phe Ser Cys Arg Leu Cys Gly Lys Leu Leu Ala Val
   225                 230                 235                 240
   Lys Gly Asp Trp Arg Thr His Glu Lys Asn Cys Gly Lys Arg Trp Val
                   245                 250                 255
   Cys Val Cys Gly Ser Asp Phe Lys His Lys Arg Ser Leu Lys Asp His
               260                 265                 270
   Val Lys Ala Phe Gly Ser Gly His Gly Pro Tyr Pro Thr Gly Leu Phe
           275                 280                 285
   Glu Glu Gln Ala Ser Asn Ser Ser Val Ser Glu Thr Leu Phe Phe
       290                 295                 300
<210> 4
   <211> 1816
   <212> DNA
   <213> *Arabidopsis thaliana*
<400> 4
   atggagtcac caccactata cgagatatcc tcaagctctt cttctgaaaa acctagacac 60
   catttccaat cccttgatct cttccctaac ctcaaccaaa actcttgtat caacaatacc 120
   ctaattgagc ctttaccgct tattgatcgc ataaacttga actcaaacct agacctaaac 180
   cctaatccct tgtatgcgga agaaggagag caagaggagg aagaagaaga agaagaagac 240
   cgtgaagtgg acgtggactt acacatcggc cttcctggtt ttggtaaacc aagcaatgat 300
   gctaaacagc tgaagaagag aaatgggaag gagatcgcca catatgacgc cggaaaaggc 360
   atcgagaatg aactttccgg aaaggcatac tggatcccgg cgccggagca aattctcata 420
   gggttcactc atttttcttg ccatgtatgc ttcaagacat tcaatcgcta caacaatctt 480
   caggtacgag tcaatatatc tcatgcgcat tgcttttcca tgcacaaaca tatataataa 540
   attcatctta tagagttata tctccggatc taatgttatg agtttattca tatctatata 600
   tatacatata tatatatata tatatatata tatatatata attctgaatt tatttgataa 660
   aagctaaaca accaggattt aatagatgat ttacctttgg atcttattat acaatttaca 720
   aatttaatca agtcaactaa tcgtgattta attacttttt tttgtaagaa gagttggtaa 780
   tatatatttt tatggtaatg ttttcatgaa aataattcat cacaactctt tacatttatt 840
   taatgcctta actaaagctg aattcgaaaa agttgaaata aattatctac taagatttga 900
   ttgactatag tttttaatag ttttcttttc tcatatatat attatcatag tagtcaaaac 960
   atttgattca aacttaaata cacagatttc ttgaatgaaa cattactatg ctcggtcaat 1020
   aatatgattt taaggaacca tgttatttca ttttattact taaggaaacc tttttgtttt 1080
   ttgttgactc taaatattat gaatatagat gcacatgtgg ggacatggtt cacaatacag 1140
   gaaaggaccg gagtcactga aaggcacaca gccacgagcc atgttaggga tcccttgtta 1200
   ctgctgcgtt gaagggtgca ggaaccacat tgaccatcct cgttccaagc cactgaaaga 1260
   ctttaggacg ctccaaacgc actacaaacg caaacacgga cacaaaccct tctcgtgtcg 1320
   cctttgcggt aagcttttgg ctgtcaaggg cgattggcga acacatgaga agaattgtgg 1380
   aaaacgttgg gtttgcgttt gcggttctga ttttaaacac aaacgttctc ttaaggacca 1440
   tgttaaggcg tttgggtctg gtcatgggcc ttatccaact ggtttgtttg aagagcaggc 1500
   ttctaattca tctgtctccg agactttgtt tttttaaatt tgggcatctt tttctttcgc 1560
   ttatgaaata tctatttact ttagaaaaat aataatgtgg tatctaattg ttccaaatta 1620
   ggaacacgaa gtgtaccatt atatttttca tcactacaaa tgttattcag agaaaattat 1680
   cattaattgt ctcgttaaag atagaatagg gtttgaattt atcaaatatt aaaaacagat 1740
   caatacaaaa ttgaccatgc atatgcactt gaatattctg atttctttat gatgtaatct 1800
   cattcaagaa aagctt                                                 1816